# EUROPÄISCHE PATENTSCHRIFT

(11) **EP 0 670 828 B1**
(45) Veröffentlichungstag und Bekanntmachung des Hinweises auf die Patenterteilung: **18.06.1997**
(21) Anmeldenummer: 94901845.1
(22) Anmeldetag: 22.11.1993
(51) Int. Cl.: C07C 301/00, C07B 45/02, C14C 9/02, C11C 3/00

(54) **SULFITIERTE FETTSTOFFE MIT VERMINDERTEM GEHALT AN FREIEM HYDROGENSULFIT**
SULPHITED FATS WITH A REDUCED FREE-BISULPHITE CONTENT
GRAISSES SULFITEES A TENEUR REDUITE EN SULFITE D'HYDROGENE LIBRE

(30) Priorität: 30.11.1992 DE 4240159
(43) Veröffentlichungstag der Anmeldung: 13.09.1995
(73) Patentinhaber: Henkel Kommanditgesellschaft auf Aktien, 40191 Düsseldorf (DE)
(72) Erfinder: SEGURA, Ramon, E-08029 Barcelona (ES)
(86) Internationale Anmeldenummer: EP9303274
(87) Internationale Veröffentlichungsnummer: WO9412471

(56) Entgegenhaltungen:
- EP-A- 0 247 490
- DE-A- 3 317 422

## Beschreibung

Die Erfindung betrifft sulfitierte Fettstoffe mit vermindertem Gehalt an freiem Hydrogensulfit, ein Verfahren zu ihrer Herstellung, bei dem man ungesättigte Fettstoffe, bläst, sulfitiert und nichtabreagiertes Hydrogensulfit durch Nachbehandlung mit Maleinsäureestern und gegebenenfalls Basen als Sulfosuccinate bindet sowie die Verwendung der sulfitierten Fettstoffe als Lederhilfsmittel.

### Stand der Technik

Neben den Gerbstoffen sind Fettungsmittel die wichtigsten Hilfsmittel, um den Charakter von Leder zu prägen. Die Wirkung der Fettungsmittel kommt durch eine faserisolierende Schmierung und durch eine Hydrophobierung zustande.

Durch Umhüllung der Lederfasern mit einem Fettfilm wird die gegenseitige Reibung verringert und demzufolge die Geschmeidigkeit und Dehnbarkeit des Gewebes verbessert. Das hat positive Auswirkungen auf die Reißfestigkeit des Leders, denn in einem dehnbaren Werkstoff richten sich viele Fasern bei Zugbeanspruchung in der Zugrichtung aus und setzen dann dem Zerreißen einen größeren Widerstand entgegen als dieselben Fasern innerhalb eines spröden Werkstoffes. Durch die Hydrophobierung werden darüber hinaus gerbende Effekte erzielt, da sie mit einer Verdrängung von Wasser aus der Haut verbunden ist.

Als Lederfettungsmittel werden im allgemeinen pflanzliche und tierische Öle, Fette und Wachse eingesetzt, ferner die aus diesen Stoffen durch chemische Umwandlung gewonnenen Hydrolyse-, Sulfierungs-, Oxidations- und Härtungsprodukte und schließlich mineralische Fettungsmittel; im einzelnen:
o Die verseifbaren Fette und Öle sowie die natürlichen Wachse und Harze gehören zu den Estern. Unter Ölen und Fetten werden dabei vom Lederfachmann Ester aus Glycerin und Fettsäuren bezeichnet, die bei Raumtemperatur fest bzw. flüssig sind. Zur Lederfettung werden dabei aus der Gruppe der tierischen Fette insbesondere Trane, Fischöl, Rindertalg und Rinderklauenöl, aus der Gruppe der pflanzlichen Fette Rizinusöl, Rüböl und Leinöl herangezogen. In Wachsen und Harzen sind die Fettsäuren statt mit Glycerin mit höhermolekularen Alkoholen verestert. Beispiele für Wachse sind Bienenwachs, chinesisches Wachs, Caranubawachs, Montanwachs und Wollfett; zu den wichtigsten Harzen zählen Kolophonium, Juchtenöl und Schellack.
o Durch chemische Umwandlung pflanzlicher und tierischer Fette erhält man Produkte, die wasserlöslich sind und die darüber hinaus in unterschiedlichem Maße emulgierend auf wasserunlösliche Fettstoffe wirken. Bekannt sind etwa die sulfierten wasserlöslichen Öle verschiedenster Art, die durch Oxidation veränderten Trane, die als Dégras oder Moellon bezeichnet werden, ferner die Seifen, die bei der hydrolytischen Spaltung natürlicher Fette entstehen, gehärtete Fette sowie schließlich freie Fettsäuren wie Stearinsäure als Einbrennfette. Die meisten tierischen und pflanzlichen Fette weisen eine gewisse Affinität zur Ledersubstanz auf, die durch die Einführung oder Freilegung hydrophiler Gruppen noch beträchtlich gesteigert wird.
o Wichtig für die Lederherstellung sind weiter die mineralischen Fettungsmittel. Diese Kohlenwasserstoffe sind den natürlichen Fetten und Ölen in manchen Eigenschaften ähnlich, lassen sich jedoch nicht verseifen. Es handelt sich um Fraktionen der Erdöldestillation, die in flüssiger Form Mineralöl, in pastöser Form Vaseline und in fester Form Paraffin genannt werden.

In vielen Fällen bilden sich jedoch auf der Oberfläche der gegerbten und gefetteten Leder im Laufe der Zeit unerwünschte Flecken, die als "Fettausschläge" (fatty spew) bezeichnet werden. Fettausschläge entstehen vornehmlich auf chromgegerbten Ledern nach kürzerer oder längerer Lagerung als weißer, oft schleierartiger Belag, der nur einzelne Stellen oder auch die ganze Lederfläche bedeckt. Der Ausschlag ist auf ein Austreten von festen Fettstoffen aus dem Leder zurückzuführen. Er kann durch das an sich im Leder vorhandene Naturfett oder durch Fettstoffe verursacht sein, die erst im Zuge der Fettung der Leder einverleibt worden sind.

Zum Fetten von Leder benutzte Fettgemische neigen insbesondere dann zur Bildung von Ausschlag, wenn sie viel freie Fettsäuren enthalten. Freie Fettsäuren weisen im allgemeinen einen höheren Schmelzpunkt auf als ihre Glyceride. Die hydrolytische Spaltung von Fettstoffen bei der Lagerung des Leders erhöht entsprechend die Gefahr des Auftretens von Fettausschlägen.

Seifen und Lickerfette werden in Chromleder, besonders in nicht genügend entsäuertem Chromleder, unter Freisetzen von Fettsäuren gespalten. Sulfierte Öle und Fette weisen eine unterschiedlich starke Neigung zur Bildung von Fettausschlägen auf, die Ausschlagsneigung geht mit längerer Lebensdauer im allgemeinen zurück [vgl. **J.Int.Soc.Leath.Trad.Chem. 379 (1952)**].

Fettausschläge treten umso leichter auf, je mehr das Leder zur Ausschlagsbildung neigende Fettstoffe enthält. Für den Umfang und die Zusammensetzung des Ausschlags sind Menge, Zusammensetzung und Lage des im Leder vorhandenen Fettgemisches aus Naturfett und Lickerfett maßgebend. Locker strukturiertes Leder neigt weniger zur Ausschlagsbildung als Leder mit dichtem Fasergefüge. Fettausschläge werden bei niedrigen Temperaturen häufiger beobachtet als bei wärmeren Außentemperaturen.

Die kristallinen Fettausschläge entwickeln sich in den Haarlöchern und Drüsenkanälen, wobei zunächst kleine Kristalle in der Tiefe gebildet werden, die allmählich als größere Fettkristalle das ganze Haarloch ausfüllen, über die Lederoberfläche hinausquellen und zu einem dichten Kristallfilm verfilzen. Alle Fette, die Stearin- oder Palmitinderivate enthalten, können kristalline Fettausschläge verursachen, mit zunehmender Konzentration wird die Ausschlagsgefahr vergrößert [**Ledertechn.Rundsch**. **1 (1949)**].

Insbesondere neigen die sogenannten Neutralfette, d.h. solche zur Lederfettung geeigneten Substanzen, die keine ionischen Gruppen im Molekül enthalten, z.B. Fette, Wachse und Kohlenwasserstoffe, zur Bildung von Fettausschlägen. Besonders kritisch sind dabei diejenigen Neutralfette, die Stearin-und/oder Palmitinderivate darstellen, wie etwa entsprechende Triglyceride oder die freien Fettsäuren.

Da im Zuge der Lederverarbeitung jedoch nach dem Gerben ohnehin als nahezu obligatorischer Arbeitsgang eine Fettung erforderlich ist, um die angestrebten Produkteigenschaften zu erreichen, ist es in der Praxis üblich geworden, mit speziellen synthetischen Fettungsmitteln zu arbeiten, deren Neigung zur Bildung von Fettausschlag gering ist.

Eine in dieser Hinsicht üblicherweise eingesetzte Klasse von Fettungsmitteln sind halogenierte Verbindungen wie Chlorkohlenwasserstoffe. Die steigenden ökologischen und toxikologischen Anfoderungen an Mittel, die in die Umwelt gelangen, bzw. mit denen der Verbraucher in Berührung kommt, machen jedoch diese Substanzklasse zunehmend unattraktiv. Die Verwendung von Chlorparaffinen als Additive zu Fettlicker-Emulsionen, um die Ausschlagsbildung auf chromgegerbtem Schweinelder zu verhindern beschreibt z.B. J.Golonka in **Przegl. Skorzany 42(2), 35** (zitiert nach Chem. Abstracts 107(18):156865z).

Aus der Europäischen Patentschrift **EP-B 0 247 509** (Stockhausen) sind Anlagerungsprodukte von Schwefelsäure bzw. Oleum an ungesättigte, alkoxylierte sowie gegebenenfalls epoxidierte Fette und Öle bekannt. Produkte dieser Art weisen jedoch in der Regel einen unvorteilhaft hohen Elektrolytgehalt auf, so daß die Gefahr besteht, daß die Salze auskristallisieren und die Qualität des behandelten Leders verschlechtern.

Neben Umsetzungen mit Oleum, Schwefelsäure oder gasförmigem Schwefeltrioxid kommt für die Hydrophilisierung von Fettstoffen insbesondere auch die Sulfitierung, d. h. die Anlagerung von Hydrogensulfit an ungesättigte Verbindungen, in Betracht. So wird beispielsweise in diesem Zusammenhang von A.Küntzel über die Sulfitierung von Dorschtran [**Leder 8, 5 (1957)**] sowie von M.Mikula über die Sulfitierung von ungesättigten Fettsäurebutylestern [**Leder, Schuh, Lederwaren 21, 282 (1986)**] berichtet. Obschon sulfitierte Fettstoffe ein besonders vorteilhaftes Fettungsverhalten aufweisen, neigen auch sie zur Bildung von unerwünschten Fettausschlägen. Ein weiterer Nachteil besteht ferner darin, daß die Produkte infolge von geringen Anteilen nichtabreagierten Hydrogensulfits einen stechenden Geruch aufweisen.

Die aus dem Stand der Technik bekannten Methoden zur Verhinderung von Fettausschlag vermögen deshalb insgesamt nicht zu befriedigen.

Aus dem geschilderten Kontext heraus ist klar, daß die Lederindustrie einen ständigen Bedarf an Additiven bzw. Fettungsmitteln hat, die Fettausschlag auf wirksame Weise verhüten und um dadurch die Palette der handelsüblichen Produkte zu ergänzen und flexibel auf die sich ändernden Anforderungen des Marktes reagieren zu können. Insbesondere besteht ein Bedarf an ökologisch bzw. toxikologisch unbedenklichen Additiven und Fettungsmitteln, die bei ihrer Anwendung nicht zu einer unerwünschten Bildung eines Fettausschlags führen.

Die Aufgabe der Erfindung bestand somit darin, neue Stoffe für die Fettung und Hydrophobierung von Leder zur Verfügung zu stellen, die frei von den geschilderten Nachteilen sind.

### Beschreibung der Erfindung

Gegenstand der Erfindung sind sulfitierte Fettstoffe mit vermindertem Gehalt an freiem Hydrogensulfit, die man erhält, indem man ungesättigte Fettstoffe in an sich bekannter Weise bläst, mit Natriummetabisulfit sulfitiert und anschließend nichtumgesetztes Hydrogensulfit durch Nachbehandlung mit Maleinsäureestern und gegebenenfalls Basen als Sulfobernsteinsäureester bindet.

Überraschenderweise wurde gefunden, daß sich der Restgehalt an freiem Hydrogensulfit in geblasenen, sulfitierten Fettstoffen signifikant auf Werte unterhalb von 1 Gew.-% - bezogen auf den Feststoffgehalt der Produkte - verringern läßt, wenn man die Rohprodukte mit Maleinsäureestern und gegebenenfalls Basen nachbehandelt. Die Erfindung beruht auf der Erkenntnis, daß das freie Hydrogensulfit mit den Maleinsäureestern unter Bildung von Sulfobernsteinsäureestern ("Sulfosuccinaten") rasch und praktisch vollständig abreagiert. Die erfindungsgemäßen sulfitierten Fettstoffe, die einen Gehalt an Sulfosuccinaten aufweisen, zeigen gegenüber üblichen Produkten des Stands der Technik deutlich verbesserte anwendungstechnische Eigenschaften. Insbesondere ist ihre Neigung zur Bildung von Fettausschlägen auf Lederoberflächen signifikant verringert; die Produkte sind zudem praktisch geruchsfrei.

Ein weiterer Gegenstand der Erfindung betrifft ein Verfahren zur Herstellung sulfitierter Fettstoffe mit vermindertem Gehalt an freiem Hydrogensulfit, bei dem man ungesättigte Fettstoffe in an sich bekannter Weise bläst, mit Natriumhydrogensulfit sulfitiert und anschließend nichtumgesetztes Hydrogensulfit durch Nachbehandlung mit Maleinsäureestern und gegebenenfalls Basen als Sulfobernsteinsäureester bindet.

### Ausgangsstoffe

Unter Fettstoffen, die als Ausgangsstoffe für die Herstellung der sulfitierten Produkte dienen, können Fettsäureglyceride, Fettsäureniedrigalkylester oder deren Gemische eingesetzt werden.
a) Unter **Fettsäureglyceriden** sind Stoffe der Formel (I) zu verstehen, 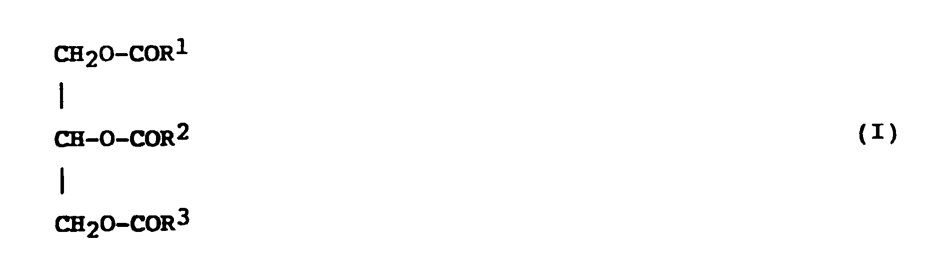 in der R¹CO, R²CO und R³CO unabhängig voneinander für aliphatische Acylreste mit 12 bis 24 Kohlenstoffatomen und 1, 2, 3 oder 4 Doppelbindungen stehen.
   Die Fettsäureglyceride können synthetischer oder insbesondere pflanzlicher bzw. tierischer Herkunft sein. Typische Beispiele sind Triglyceride, die als Fettsäurekomponenten ganz oder überwiegend Palmoleinsäure, Ölsäure, Elaidinsäure, Petroselinsäure, Linolsäure, Linolensäure, Gadoleinsäure, Erucasäure, Arachidonsäure und/ oder Clupanodonsäure enthalten. Als bevorzugte Ausgangsstoffe kommen in diesem Zusammenhang Glycerintrioleat sowie pflanzliche oder tierische Fette und Öle in Betracht, die eine Iodzahl im Bereich von 50 bis 150, vorzugsweise 80 bis 140 aufweisen. Ohne Anspruch auf Vollständigkeit sollen dabei Olivenöl, Olivenkernöl, Sonnenblumenöl alter und neuer Züchtung, Rapsöl alter und neuer Züchtung, Baumwollsaatöl, Erdnußöl, Korianderöl, Leinöl, Rindertalg und Fischöl genannt werden. Der Natur entsprechend, können die pflanzlichen bzw. tierischen Fette und Öle auch gesättigte Anteile aufweisen, sofern diese 50 Gew.-% nicht übersteigen.
b) Unter **Fettsäureniedrigalkylestern** sind Stoffe der Formel (II) zu verstehen,

   **R**^{**4**}**CO-OR**^{**5**} **(II)**

   in der R⁴CO für aliphatische Acylreste mit 12 bis 24 Kohlenstoffatomen und 1, 2, 3 oder 4 Doppelbindungen und R⁵ für lineare oder verzweigte Alkylreste mit 1 bis 4 Kohlenstoffatomen steht.
   Typische Beispiele sind Ethyl-, Propyl-, Butyl- und insbesondere Methylester der Palmoleinsäure, Ölsäure, Elaidinsäure, Petroselinsäure, Linolsäure, Linolensäure, Gadoleinsäure, Erucasäure, Arachidonsäure und/oder Clupanodonsäure sowie deren technischen Mischungen, wie sie beispielsweise bei der Druckspaltung der vorgenannten pflanzlichen und tierischen Fette und Öle anfallen. Vorzugsweise werden Öl- und/oder Linolsäuremethylester eingesetzt.

Wie bereits oben ausgeführt, können die Fettsäureglyceride und die Fettsäureniedrigalkylester sowohl alleine als auch in Mischung eingesetzt werden. Als besonders vorteilhaft hat es sich jedoch erwiesen, Gemische von ungesättigten Fettsäureglyceriden und ungesättigten Fettsäureniedrigalkylestern gemeinsam zu sulfitieren. Das molare Mischungsverhältnis ist dabei in weiten Bereichen unkritisch und kann beispielsweise 1 : 5 bis 5 : 1, vorzugsweise 1 : 1 bis 3 : 1 betragen.

### Blasen und Sulfitierung

Das Blasen von Fettstoffen stellt ein bekanntes Verfahren dar. Man versteht hierunter das Einbringen von (Luft-)Sauerstoff bei 100 bis 150°C in ungesättigte Fettstoffe. Ziel ist es, durch teilweise Autoxidation die Dickflüssigkeit und Beständigkeit der Stoffe zu erhöhen [vgl. **ROEMPP Chemielexikon, Thieme Verlag, Stuttgart, 9.Aufl., Bd.II, S.1498 (1990)**]. Im Sinne des erfindungsgemäßen Verfahrens hat es sich als vorteilhaft erwiesen, die Fettstoffe bei 100 bis 110°C solange zu blasen, bis eine Dichtezunahme um 0,01 bis 0,1, vorzugsweise 0,02 bis 0,05 g/cm³ erreicht wird.

Die Sulfitierung kann in an sich bekannter Weise durchgeführt werden, wobei man Natriumhydrogensulfit an die Doppelbindung der ungesättigten Fettstoffe anlagert. Da Natriumhydrogensulfit nur in wäßriger Lösung beständig ist, wird als Sulfitierungsmittel Natriummetabisulfit (Na₂SO₃) eingesetzt. Üblicherweise können die ungesättigten Fettstoffe und das Natriummetabisulfit im molaren Verhältnis von 3 : 1 bis 15 : 1, vorzugsweise 5 : 1 bis 10 : 1 - bezogen auf die Doppelbindungsäquivalente in den ungesättigten Fettstoffen - eingesetzt. werden. Als optimal hat es sich erwiesen, die Sulfitierung über einen Zeitraum von 1 bis 5, vorzugsweise 2 bis 4 h bei Temperaturen von 50 bis 120, vorzugsweise 60 bis 90°C durchzuführen.

### Nachbehandlung

Im Sinne der Erfindung wird bei der Sulfitierung nichtabreagiertes Hydrogensulfit durch Nachbehandlung mit einem Maleinsäureester als Sulfobernsteinsäureester ("Sulfosuccinat") gebunden.

Als **Maleinsäureester** kommen Stoffe der Formel **(IV)** in Betracht,

**R**^{**7**}**COO-CH=CH-COOR**^{**8**} **(IV)**

in der R⁷ für einen Alkyl- und/oder Alkenylrest mit 6 bis 22 Kohlenstoffatomen und R⁸ für Wasserstoff oder einen Alkyl- und/oder Alkenylrest mit 6 bis 22 Kohlenstoffatomen steht. Typische Beispiele sind Mono- oder Diester der Maleinsäure bzw. des Maleinsäureanhydrids mit Capronalkohol, Caprylalkohol, Caprinalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachinyl-alkohol, Gadoleylalkohol, Behenylalkohol und Erucylalkohol sowie deren technischen Gemischen, wie sie beispielsweise bei der Hydrierung von technischen Methylesterfraktionen oder Aldehyden aus der Roelen'schen Oxosynthese anfallen. Vorzugsweise werden Maleinsäuremonoester von technischen Kokos-oder Talgfettalkoholen und insbesondere Oleylalkohol eingesetzt.

Die Maleinsäureester können in Mengen von 10 bis 100 Mol-% - bezogen auf die Menge an eingesetztem Natriummetabisulfit - eingesetzt werden. Üblicherweise wird man die Menge so wählen, daß sie dem Restgehalt an freiem Hydrogensulfit entspricht; demzufolge stellt ein Mengenbereich von 15 bis 30 Mol-% - wieder bezogen auf die Menge an eingesetztem Metabisulfit - eine bevorzugte Auswahl dar. Im Hinblick auf die anwendungstechnischen Eigenschaften des Produktes kann es jedoch sein, daß die durch die Abreaktion des freien Hydrogensulfits mit dem Maleinsäureester erreichte Sulfosuccinat-Konzentration nicht ausreichend ist. In diesem Fall kann der Anteil an freiem Metabisulfit im rohen Sulfitierungsprodukt künstlich, beispielsweise von 15 auf 35 Gew.-% - bezogen auf den Feststoffgehalt des Produktes - aufgestockt werden. Diesem Umstand wird der Fachmann bei der Auswahl der Maleinsäureester-Menge gewohnheitsmäßig Rechnung tragen können, ohne dazu erfinderisch tätig werden zu müssen.

Die Nachbehandlung der rohen Sulfitierungsprodukte mit den Maleinsäureestern kann in Gegenwart von wäßrigen Basen, wie beispielsweise Alkali- bzw. Erdalkalihydroxiden oder Ammoniak erfolgen. Nachdem es die Aufgabe der Base ist, freie Carboxylgruppen der Maleinsäure- bzw. Sulfobernsäureester - sofern vorhanden - zu neutralisieren, wird man deren Menge mit Bedacht so auswählen, daß sich im Produkt ein pH-Wert einstellt, bei dem eine Verseifung der Esterbindungen nicht zu befürchten ist.

Die Nachbehandlung kann über einen Zeitraum von 0,5 bis 5, vorzugsweise 1 bis 3 h bei Temperaturen von 50 bis 90, vorzugsweise 70 bis 85°C durchgeführt werden.

### Gewerbliche Anwendbarkeit

Die sulfitierten Fettstoffe verleihen Leder eine angenehme Geschmeidigkeit und eine Resistenz gegenüber Feuchtigkeit. Sie sind praktisch geruchsfrei, neigen nicht zur Bildung von Fettausschlägen und sind leicht biologisch abbaubar. Da freies Hydrogensulfit nicht auf dem Leder haftet, sondern nach der Fettung ausgewaschen wird, stellt der vergleichsweise geringere Elektrolytgehalt der erfindungsgemäßen sulfitierten Fettstoffe unter ökologischen Gesichtspunkte einen zusätzlichen Vorteil dar.

Ein weiterer Gegenstand der Erfindung betrifft daher Lederfettungsmittel, insbesondere für chromgegerbtes Leder, die einen Gehalt der erfindungsgemäßen sulfitierten Fettstoffe von 1 bis 99, vorzugsweise 10 bis 50 Gew.-% aufweisen können. Neben den erfindungsgemäßen Stoffen können diese Fettungsmittel weitere übliche Zusatzstoffe, wie beispielsweise Fettsäureester und Dialkylether aufweisen.

Ein weiterer Gegenstand.der Erfindung betrifft schließlich die Verwendung der erfindungsgemäßen sulfitierten Fettstoffe zur Herstellung von Fettungs- und Hydrophobiermittel für insbesondere chromgegerbtes Leder, in denen sie in Mengen von 1 bis 99, vorzugsweise 10 bis 50 Gew.-% enthalten sein können.

Die folgenden Beispiele sollen den Gegenstand der Erfindung näher erläutern, ohne ihn darauf einzuschränken.

### Beispiele

### Beispiel 1:

In einem 2-1-Dreihalskolben mit Rührer, Rückflußkühler und Tropftrichter wurde eine Mischung aus 250 g (0,2 mol) Fischöl (Verseifungszahl 130, Iodzahl 145) und 500 g (0,55 mol) Olivenkernöl (Verseifungszahl 185, Iodzahl 95) vorgelegt und bei 105°C mit Luft geblasen, bis eine Dichtezunahme von 0,03 g/cm³ (gemessen bei 20°C) erzielt wurde. Anschließend wurde die Mischung auf 60°C abgekühlt und über den Tropftrichter portionsweise eine Lösung von 90 g (0,9 mol) Natriummetabisulfit in 170 ml entsalztem Wasser zudosiert. Nach Beendigung der Zugabe wurde die Reaktionsmischung auf 85°C erhitzt und bei dieser Temperatur weitere 2 h gerührt. Danach wurde nicht abreagiertes Hydrogensulfit durch den Zusatz von 75 g (0,2 mol) Maleinsäure-mono-oleylester und 20 g (0,85 mol) Ammoniak in Form einer 50 gew.-%igen wäßrigen Lösung als Sulfobernsteinsäuremonoester gebunden. Der Restgehalt an freiem Hydrogensulfit betrug 0,5 Gew.-% bezogen auf den Feststoffgehalt des sulfitierten Produktes.

### Beispiel 2:

Analog Beispiel 1 wurde eine Mischung aus 250 g (0,2 mol) Fischöl, 250 g (0,28 mol) Glycerintrioleat und 250 g (0,84 mol) Olivenkernfettsäuremethylester wiederum bei 105°C geblasen, bis eine Dichtezunahme von 0,03 g/cm³ erzielt wurde und anschließend mit 68 g (0,68 mol) Natriummetabisulfit in 120 ml Wasser sulfitiert. Nichtumgesetztes Hydrogensulfit sowie weitere 22 g (0,22 mol) Natriummetabisulfit, die dem abreagierten Reaktionsgemisch zugesetzt wurden, wurden anschließend durch Zugabe von 75 g (0,2 mol) Maleinsäure-mono-oleylester und 20 g (0,85 mol) Ammoniak in Form einer 25 gew.-%igen wäßrigen Lösung als Sulfobernsteinsäuremonoester gebunden. Der Restgehalt an freiem Hydrogensulfit betrug 0,9 Gew.- % bezogen auf den Feststoffgehalt des sulfitierten Produktes.

## Patentansprüche

1. Sulfitierte Fettstoffe mit vermindertem Gehalt an freiem Hydrogensulfit, dadurch erhältlich, daß man ungesättigte Fettstoffe in an sich bekannter Weise bläst, mit Natriummetabisulfit sulfitiert und anschließend nichtumgesetztes Hydrogensulfit durch Nachbehandlung mit Maleinsäureestern und gegebenenfalls Basen als Sulfobernsteinsäureester bindet.

2. Verfahren zur Herstellung sulfitierter Fettstoffe mit vermindertem Gehalt an freiem Hydrogensulfit, bei dem man ungesättigte Fettstoffe in an sich bekannter Weise bläst, mit Natriummetabisulfit sulfitiert und anschließend nichtumgesetztes Hydrogensulfit durch Nachbehandlung mit Maleinsäureestern und gegebenenfalls Basen als Sulfobernsteinsäureester bindet.

3. Verfahren nach Anspruch 2, **dadurch gekennzeichnet,** daß man als Fettstoffe Fettsäureglyceride und/oder Fettsäureniedrigalkylester einsetzt.

4. Verfahren nach Anspruch 2, **dadurch gekennzeichnet,** daß man als Fettstoffe Fettsäureglyceride der Formel **(I)** einsetzt, 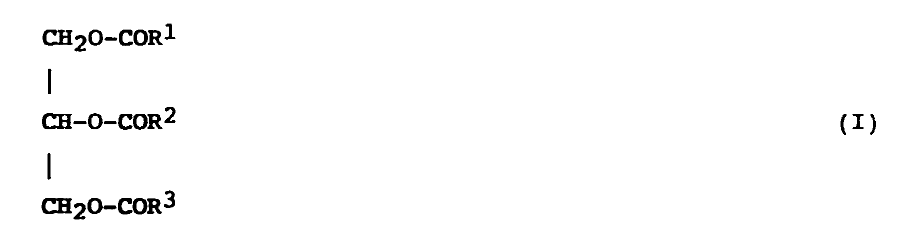 in der R¹CO, R²CO und R³CO unabhängig voneinander für aliphatische Acylreste mit 12 bis 24 Kohlenstoffatomen und 1, 2, 3 oder 4 Doppelbindungen stehen.

5. Verfahren nach Anspruch 2, **dadurch gekennzeichnet,** daß man als Fettstoffe Fettsäureniedrigalkylester der Formel **(II)** einsetzt,
**R**^{**4**}**CO-OR**^{**5**} **(II)**
in der R⁴CO für aliphatische Acylreste mit 12 bis 24 Kohlenstoffatomen und 1, 2, 3 oder 4 Doppelbindungen und R⁵ für lineare oder verzweigte Alkylreste mit 1 bis 4 Kohlenstoffatomen steht.

6. Verfahren nach Anspruch 2, **dadurch gekennzeichnet,** daß man die ungesättigten Fettstoffe durch Einbringen von Luftsauerstoff behandelt ("bläst"), bis eine Dichtezunahme von 0,01 bis 0,1 g/cm³ erzielt wird.

7. Verfahren nach Anspruch 2, **dadurch gekennzeichnet,** daß man die ungesättigten Fettstoffe und das Natriummetabisulfit im molaren Verhältnis von 3 : 1 bis 15 : 1 - bezogen auf die Doppelbindungsäquivalente in den ungesättigten Fettstoffen - einsetzt.

8. Verfahren nach Anspruch 2, **dadurch gekennzeichnet,** daß man Maleinsäureester der Formel **(IV)** einsetzt,
**R**^{**7**}**COO-CH=CH-COOR**^{**8**} **(IV)**
in der R⁷ für einen Alkyl- und/oder Alkenylrest mit 6 bis 22 Kohlenstoffatomen und R⁸ für Wasserstoff oder einen Alkyl- und/oder Alkenylrest mit 6 bis 22 Kohlenstoffatomen steht.

9. Verfahren nach Anspruch 2, **dadurch gekennzeichnet,** daß man die Maleinsäureester in Mengen von 10 bis 100 Mol-% - bezogen auf die Menge an eingesetztem Natriummetabisulfit - einsetzt.

10. Verfahren nach Anspruch 2, **dadurch gekennzeichnet,** daß man als Basen Alkali- bzw. Erdalkalihydroxide oder Ammoniak in Form wäßriger Lösungen einsetzt.

11. Verfahren nach Anspruch 2, **dadurch gekennzeichnet,** daß man die Nachbehandlung bei Temperaturen von 50 bis 90°C durchführt.

12. Lederfettungsmittel mit einem Gehalt an sulfitierten Fettstoffen nach Anspruch 1.

13. Verwendung von sulfitierten Fettstoffen nach Anspruch 1 zur Herstellung von Fettungs- und Hydrophobiermitteln für chromgegerbte Leder.

## Claims

1. Sulfited fatty compounds with a reduced content of free hydrogen sulfite obtainable by blowing unsaturated fatty compounds in known manner and sulfiting the blown fatty compounds with sodium metabisulfite and subsequently fixing unreacted hydrogen sulfite as sulfosuccinic acid ester by aftertreatment with maleic acid esters and optionally bases.

2. A process for the production of sulfited fatty compounds with a reduced content of free hydrogen sulfite, in which unsaturated fatty compounds are blown in known manner and sulfited with sodium metabisulfite, after which unreacted hydrogen sulfite is fixed as sulfosuccinic acid ester by aftertreatment with maleic acid esters and optionally bases.

3. A process as claimed in claim 2, **characterized in that** fatty acid glycerides and/or fatty acid lower alkyl esters are used as the fatty compounds.

4. A process as claimed in claim 2, **characterized in that** fatty acid glycerides corresponding to formula **(I)**: 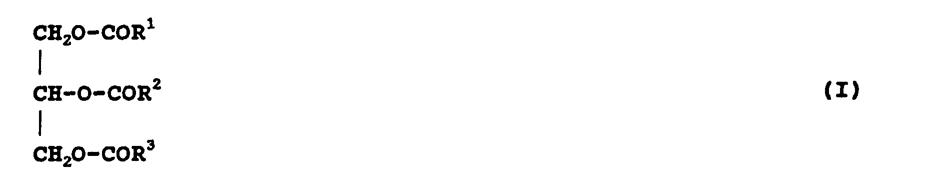 in which R¹CO, R²CO and R³CO independently of one another represent aliphatic acyl radicals containing 12 to 24 carbon atoms and 1, 2, 3 or 4 double bonds, are used as the fatty compounds.

5. A process as claimed in claim 2, **characterized in that** fatty acid lower alkyl esters corresponding to formula **(II)**:
**R**^{**4**}**CO-OR**^{**5**} **(II)**
in which R⁴CO represents aliphatic acyl radicals containing 12 to 24 carbon atoms and 1, 2, 3 or 4 double bonds and R⁵ represents linear or branched alkyl radicals containing 1 to 4 carbon atoms, are used as the fatty compounds.

6. A process as claimed in claim 2, **characterized in that** the unsaturated fatty compounds are treated by introduction of atmospheric oxygen ("blown") until an increase in density of 0.01 to 0.1 g/cm³ is obtained.

7. A process as claimed in claim 2, **characterized in that** the unsaturated fatty compounds and the sodium metabisulfite are used in a molar ratio of 3:1 to 15:1, based on the double bond equivalents in the unsaturated fatty compounds.

8. A process as claimed in claim 2, **characterized in that** maleic acid esters corresponding to formula **(IV)**:
**R**^{**7**}**COO-CH=CH-COOR**^{**8**} **(IV)**
in which R⁷ is an alkyl and/or alkenyl radical containing 6 to 22 carbon atoms and R⁸ is hydrogen or an alkyl and/or alkenyl radical containing 6 to 22 carbon atoms, are used.

9. A process as claimed in claim 2, **characterized in that** the maleic acid esters are used in quantities of 10 to 100 mole-%, based on the quantity of sodium metabisulfite used.

10. A process as claimed in claim 2, **characterized in that** alkali metal or alkaline earth metal hydroxides or ammonia in the form of aqueous solutions are used as the bases.

11. A process as claimed in claim 2, **characterized in that** the aftertreatment is carried out at temperatures of 50 to 90°C.

12. Leather oiling compositions containing the sulfited fatty compounds claimed in claim 1.

13. The use of the sulfited fatty compounds claimed in claim 1 for the production of oiling and hydrophobicizing compositions for chrome-tanned leather.

## Revendications

1. Graisses sulfitées à teneur réduite en sulfite d'hydrogène, obtenues en soufflant des corps gras insaturés de manière connue, en les sulfitant avec du métabisulfite de sodium et en liant le sulfite d'hydrogène non transformé comme ester d'acide sulfosuccinique par traitement subséquent avec des esters d'acide maléique et, le cas échéant, avec des bases.

2. Procédé de préparation de graisses sulfitées à teneur réduite en sulfite d'hydrogène libre, où les corps gras sont soufflés de manière connue en elle-même, où ils sont sulfités avec du métabisulfite de sodium et où ensuite le sulfite d'hydrogène non transformé est lié comme ester d'acide sulfosuccinique par traitement subséquent avec des esters d'acide maléique et, le cas échéant, avec des bases.

3. Procédé selon la revendication 2, caractérisé en ce que l'on utilise comme corps gras des glycérides d'acides gras et/ou des alkylesters d'acides gras à chaîne courte.

4. Procédé selon la revendication 2, caractérisé en ce que l'on utilise comme corps gras des glycérides d'acides gras de formule (I), 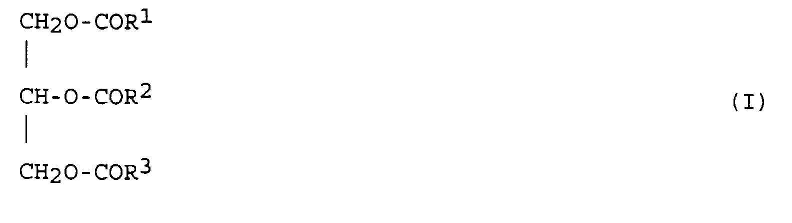 dans laquelle R¹CO, R²CO et R³CO représentent, indépendamment entre eux, des radicaux acyles aliphatiques avec de 12 à 24 atomes de carbone et 1, 2, 3 ou 4 doubles liaisons.

5. Procédé selon la revendication 2,
caractérisé en ce que
l'on utilise comme corps gras des alkylesters à chaîne courte d'acides gras de formule
R⁴CO-OR⁵ (II)
où R⁴CO représente des radicaux acyles aliphatiques avec de 12 à 24 atomes de carbones et 1, 2, 3 ou 4 doubles liaisons et R⁵ représente des radicaux alkyles linéaires ou ramifiés avec de 1 à 4 atomes de carbones.

6. Procédé selon la revendication 2,
caractérisé en ce que
les corps gras insaturés sont traités par introduction d'oxygène de l'air (« soufflés ») jusqu'à l'obtention d'une augmentation de la densité de 0,01 à 0,1 g/cm³.

7. Procédé selon la revendication 2,
caractérisé en ce que
les corps gras insaturés et le métabisulfite de sodium sont utilisés en rapport molaire de 3:1 à 15:1 -par rapport aux équivalents en doubles liaisons des corps gras insaturés-.

8. Procédé la selon revendication 2,
caractérisé en ce qu'
un ester d'acide maléique de formule (IV) est utilisé
R⁷COO-CH=CH-COOR⁸ (IV)
où R⁷ représente un radical alkyle ou alkényle avec de 6 à 22 atomes de carbone et R⁸ représente un hydrogène ou un radical alkyle ou alkényle avec de 6 à 22 atomes de carbone.

9. Procédé selon la revendication 2,
caractérisé en ce que
l'ester d'acide maléique est utilisé en quantité de 1 à 100 % en mol -par rapport à la quantité de métabisulfite de sodium employée-.

10. Procédé selon la revendication 2, caractérisé en ce que l'on utilise comme bases des hydroxydes d'alcalins ou d'alcalino terreux ou de l'ammoniaque sous forme de solutions aqueuses.

11. Procédé selon la revendication 2,
caractérisé en ce que
l'on réalise le traitement subséquent à des températures de 50 à 90°C.

12. Agents de nourriture pour cuirs, à teneur en graisses sulfitées selon la revendication 1.

13. Utilisation de graisses sulfitées selon la revendication 1 pour la préparation d'agents de nourriture et d'imperméabilisation pour des cuirs tannées au chrome.
